# FASCICULE DE BREVET EUROPEEN

(11) **EP 0 616 528 B1**
(45) Date de publication et mention de la délivrance du brevet: **17.12.1997**
(21) Numéro de dépôt: 93924038.8
(22) Date de dépôt: 13.10.1993
(51) Int. Cl.: A61K 9/50, C12N 11/04

(54) **CAPSULE IMPLANTABLE**
IMPLANTIERBARE KAPSEL
IMPLANTABLE CAPSULE

(30) Priorité: 14.10.1992 FR 9212553
(43) Date de publication de la demande: 28.09.1994
(73) Titulaire: HOSPAL INDUSTRIE, F-69883 Meyzieu Cédex (FR); INSTITUT NATIONAL DE LA SANTE ET DE LA RECHERCHE MEDICALE (INSERM), F-75654 Paris Cédex 13 (FR)
(72) Inventeur: HONIGER, Jiri, F-94800 Villejuif (FR); MUSCAT, Eric, F-69100 Villeurbanne (FR)
(86) Numéro de dépôt international: EP9302825
(87) Numéro de publication internationale: WO9408570

(56) Documents cités:
- EP-A- 0 100 285
- EP-A- 0 188 309
- WO-A-91/10425
- BIOMATERIALS vol. 13, no. 1 , Janvier 1992 , GUILDFORD (GB) pages 44 - 49 XP247075 L. KESSLER ET AL. 'diffusion properties of an artificial membrane used for langerhans islets encapsulation: an in vitro test'
- Transplantation Proceedings, Vol. 27, No. 6 (December), 1995, pp. 3393-3395; P. Prevost et al., "Application of AN69 Hydrogel to Islet Encapsulation: Evaluation in the Streptozotocin-Induced Diabetic Rat Model"

## Description

La présente invention concerne le domaine technique de l'encapsulation d'une substance. Plus précisément, la présente invention a pour objet une capsule constituée par une substance entourée d'une membrane biocompatible sélectivement perméable.

Le domaine de l'encapsulation connaît un essor important depuis un certain nombre d'années. En effet, l'encapsulation trouve une application pour la fabrication de médicaments retards pour lesquels, en choisissant la nature et la taille de la capsule, on peut déterminer le temps à partir duquel le principe actif sera libéré ainsi que sa cinétique de libération.

Les techniques d'encapsulation trouvent une application particulière dans le domaine de la transplantation de cellules ou de groupes de cellules, tels que les îlots de Langerhans. L'implantation d'îlots de Langerhans est réalisée chez des diabétiques pour pallier leur insuffisance de production en insuline. Dans le cas où les îlots sont implantés chez l'homme sans encapsulation préalable, les risques de rejet sont importants malgré les traitements immunosuppressifs. Aussi préfère-t-on encapsuler les îlots avant leur implantation, ce qui permet de former autour des îlots une enveloppe constituant une barrière antigénique ; on peut ainsi utiliser des îlots de diverses origines et notamment des îlots de porcs.

La capsule utilisée doit avoir une enveloppe imperméable aux éléments du système immunitaire, mais perméable aux nutriments nécessaires pour nourrir les îlots ainsi qu'à l'insuline sécrétée en réponse au taux de glucose dans le sang. Elle doit en outre être biocompatible et ne pas provoquer de réaction défavorable des tissus constituant le sîte d'implantation.
Diverses méthodes ont été proposées dans l'état de la technique pour produire des capsules de matériel biologique.

Ainsi, l'article de Dupuy, Gin, Baquey et Ducassou intitulé "In situ polymerization of a microencapsulating medium round living cells" publié en 1988 dans le "Journal of Biomedical Materials Research" Vol. 22 p : 1061-1070, décrit un procédé de microencapsulation de cellules vivantes. Selon ce procédé, les cellules à encapsuler sont mises en solution dans de l'agarose et extrudées dans de l'huile de paraffine. Cette extrusion est effectuée au moyen de l'aiguille d'une seringue à l'intérieur de laquelle se trouvent des cellules en suspension dans l'agarose. Cette aiguille est elle-même disposée à l'intérieur d'un tube capillaire de diamètre légèrement supérieur, à l'intérieur duquel est pompée de l'huile de paraffine. Le débit de l'huile de paraffine est maintenu plus élevé que celui de l'agarose. A l'extrêmité du capillaire, les billes sont injectées dans un bain d'huile de paraffine, refroidi à 4° C pour obtenir une gélification rapide des billes.

Les billes obtenues ici sont ensuite soumises à une seconde opération d'enrobage au moyen d'une solution polymérisable. Les billes sont aspirées dans un tube capillaire en polyéthylène. Une pièce en T est connectée à ce tube afin d'y introduire une solution de monomères (30 % acrylamide et 1,5 % bisacrylamide) auxquels on ajoute un sensibilisateur et un catalyseur. Le tube traverse alors une chambre d'irradiation à l'intérieur de laquelle est disposée une lampe à mercure haute pression permettant d'initier la polymérisation. Les billes produites sont ensuite recueillies dans un réservoir contenant un milieu de conservation.

Un tel procédé présente cependant de nombreux inconvénients et notamment celui d'être discontinu avec deux étapes bien différenciées.

D'autre part, la réaction de polymérisation présente un grand risque de toxicité pour les cellules encapsulées, et n'est applicable qu'à un certain nombre de solutions polymère.

L'article intitulé "Diffusion properties of an artificial membrane used for Langerhans islets encapsulation: an in vitro test" (Biomaterials 1992, Vol. 13 No.1) décrit l'encapsulation d'îlots de Langerhans dans une membrane fabriquée à partir d'un copolymère d'acrylonitrile et de methallyle sulfonate de sodium, utilisée pour la dialyse rénale.

Cependant le résultat d'expériences menées in vitro et in vivo décrits dans cet article ne semblent pas satisfaisants car la membrane, lorsqu'elle est placée dans la cavité péritonéale ou lorsqu'elle est au contact d'un liquide biologique (solution de sérum de veau foetal) perd environ 50 % de sa perméabilité vis-à-vis du glucose et de l'insuline. Cette baisse de perméabilité serait due à un dépôt de protéines. Or, la perméabilité vis-à-vis du glucose et de l'insuline est une caractéristique clef pour un matériau utilisé pour encapsuler des îlots de Langerhans, puisque les îlots sécrètent de l'insuline lorsqu'ils sont stimulés par du glucose.

L'invention a pour but une capsule implantable dont la substance biologiquement active conserve sa viabilité et sa fonctionnalité lorsqu'elle est implantée.

Un autre but de l'invention est de une capsule implantable, biocompatible, dont l'implantation ne suscite pas de réactions indésirables de la part des tissus au lieu de l'implantation.

Pour atteindre ces but, on propose, conformément à l'invention, une capsule implantable comprenant :
- une substance biologiquement active ;
- une enveloppe entourant la substance biologiquement active, l'enveloppe étant constituée par un hydrogel d'acrylonitrile et de méthallylsulfonate de sodium contenant au moins 80% d'eau.

La présente invention sera mieux comprise à la lecture de la description qui va suivre, en référence aux figures qui illustrent de façon schématique et sans échelle déterminée un mode d'obtention de l'objet de l'invention.
La figure 1 est un schéma de l'ensemble d'un dispositif d'obtention d'une capsule selon la présente invention.
La figure 2 est un agrandissement de la portion encadrée de la figure 1.
La figure 3 illustre une première étape d'un procédé d'obtention d'une capsule selon l'invention.
La figure 4 illustre une seconde étape d'un procédé d'obtention d'une capsule selon l'invention.
La figure 5 illustre une troisième étape d'un procédé d'obtention d'une capsule selon l'invention.
La figure 6 illustre les résultats d'un essai réalisé avec les produits de l'invention.

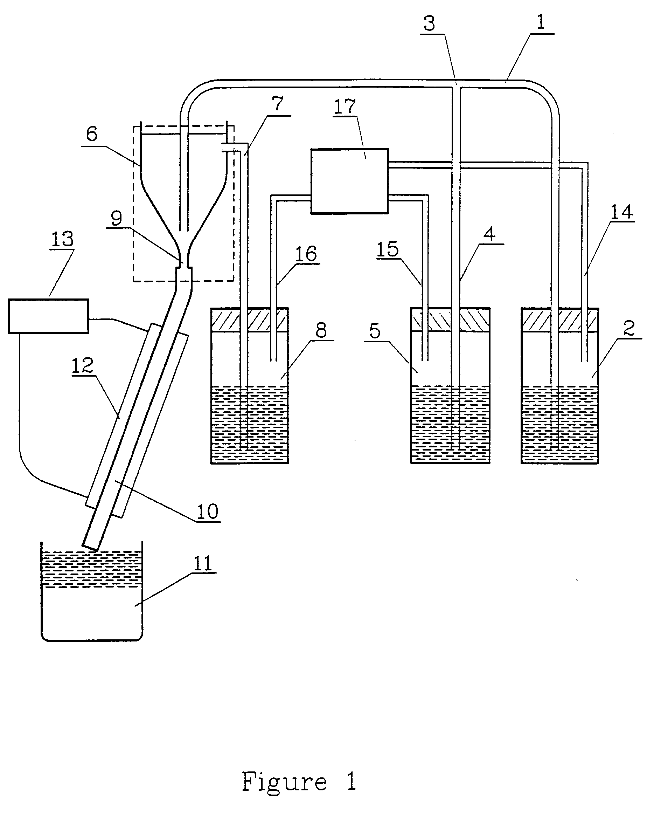

Le dispositif représenté sur la figure 1 comporte un conduit capillaire 1 d'amenée de la substance à encapsuler, présente dans le réservoir 2. Ce conduit 1 comporte une intersection 3 avec un conduit 4 véhiculant un fluide vecteur provenant du réservoir 5. L'intersection 3 qui est représentée ici comme une simple jonction de deux conduits est en réalité constituée par un dispositif d'extrusion axiale de la substance à encapsuler présente dans le conduit 1 et de coextrusion annulaire du fluide vecteur présent dans le conduit 4. Le conduit 1 débouche ensuite dans la partie centrale d'une buse 6 de forme sensiblement conique, alimentée grâce à un conduit 7 en solution polymère à partir d'un réservoir 8. La buse conique 6 est prolongée par un court canal de constriction 9 se prolongeant lui-même par un conduit réfrigéré 10 dont l'extrêmité opposée au canal de constriction 9 affleure la surface d'un liquide présent dans un réservoir 11.

Une double enveloppe 12 reliée à un dispositif de réfrigération 13, entoure le conduit 10 permettant ainsi d'abaisser la température de son contenu. Les contenus des réservoirs 2, 5 et 8 sont mis en circulation respectivement dans les conduits 1, 4 et 7, grâce à une pression exercée par de l'air comprimé amené par respectivement les canalisations 14, 15 et 16. Un dispositif 17 permet de commander l'alimentation en air de chacun des réservoirs, ce qui permet de commander leur pression et par conséquent le débit d'écoulement à l'intérieur des conduits 1, 4 et 7.

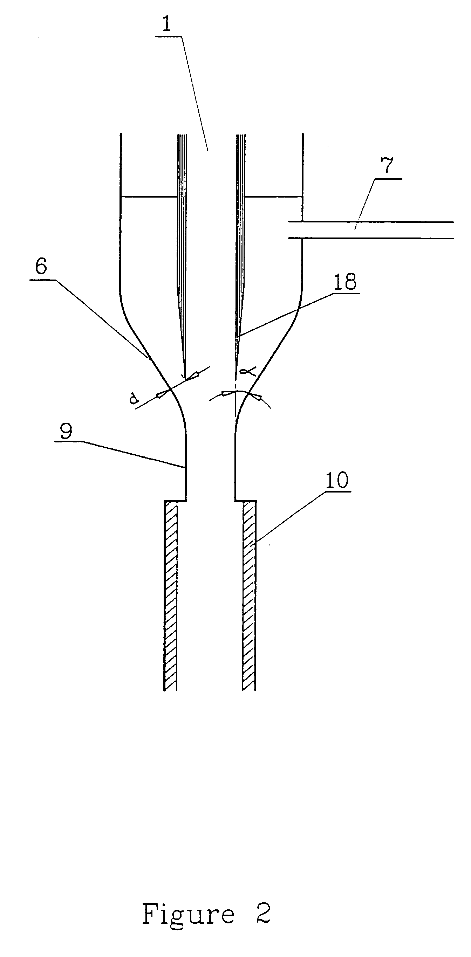

Ainsi que cela est représenté sur la figure 2, le conduit capillaire 1 présente à l'extrêmité débouchant dans la buse conique 6, une paroi effilée 18. La paroi 18 est située à une distance d de la buse conique dont l'angle d'ouverture est α. La distance d peut varier grâce à une mobilité du tube capillaire 1, à l'intérieur de la buse 6, ce qui permet de faire varier la quantité de solution polymère extrudée et par conséquent l'épaisseur de la capsule formée.

L'ensemble des conduits 1, 4, 7, 9 et 10 est constitué par un matériau lyophobe et inerte vis-à-vis des différents fluides qui y circulent. Il s'agit par exemple de polytétrafluoroéthylène (PTFE) qui présente l'avantage d'être translucide.

La substance à encapsuler peut être de différentes natures. On peut par exemple encapsuler une substance à activité biologique (telle qu'un médicament) présente dans le réservoir 2 sous forme de solution (insuline par exemple) ou de suspension ; il est également possible d'encapsuler des cellules ou groupes de cellules en suspension dans un milieu adapté à la survie cellulaire : bactéries, cellules RIN, hépatocytes, cellules pancréatiques β, îlots de Langerhans, globules rouges, cellules L929 (fibroblastes de souris), etc.

La solution polymère utilisée pour l'encapsulation comporte :
- un copolymère d'acrylonitrile et de méthallylsulfonate de sodium,
- un solvant appoprié, tel que notamment le NN-diméthylformamide (DMF), le diméthylsulfoxide (DMSO), la N-méthylpyrrolidone (NMP) ou le propylène carbonate (PC),
- un non solvant tel qu'une solution saine aqueuse.

Les proportions respectives de chacun des éléments composant la solution peuvent varier selon les caractéristiques attendues pour l'enveloppe de la capsule, notamment en ce qui concerne sa perméabilité.

Le fluide vecteur présent dans le récipient 5 est utilisé pour segmenter le flux de substance à encapsuler. On choisit à cette fin, un fluide hydrophobe, non solvant et non coagulant du polymère ou copolymère présent dans la solution polymère ; ce fluide ne doit pas être agressif envers la substance à encapsuler ; sa nature est choisie pour que les interfaces avec la substance à encapsuler d'une part et avec la solution polymère d'autre part présentent des propriétés de mouillabilité adaptées à l'encapsulation.

Un exemple de fluide vecteur convenant aux fins de la présente invention est constitué par une huile de polydiméthylsiloxane partiellement hydroxylée, le degré d'hydroxylation étant défini en fonction du degré d'hydrophilie recherché : plus le degré d'hydrophilie sera important, plus la taille des segments de substance à encapsuler sera importante.

La viscosité du fluide vecteur doit être adaptée au débit d'écoulement souhaité ainsi qu'à la taille voulue pour les segments de substance à encapsuler. En effet, toutes choses étant égales par ailleurs, lorsque la viscosité du fluide vecteur augmente, la taille des segments de substance à encapsuler diminue car la force de cisaillement augmente également.

Le liquide présent dans le réservoir 11 est destiné à réaliser l'échange du solvant contenu dans la solution polymère par un non solvant. Cette opération permet d'obtenir autour de la substance à encapsuler une enveloppe à l'état de gel non thermoréversible. Le choix du non solvant utilisé est fonction de la nature de la solution polymère extrudée. Il est avantageusement possible d'utiliser un bain comportant deux liquides dont le pouvoir de précipitation du polymère ou copolymère présent dans la solution polymère est différent. Ainsi par exemple, le bain présent dans le réservoir 11 peut être composé par une phase inférieure constituée par un non solvant fort et une phase supérieure constituée par un non solvant doux.

Par non solvant fort, on entend un non solvant qui, lorsqu'il est utilisé comme coagulant, précipite très rapidement la solution au contact de laquelle il est porté.
Au contraire, un non solvant doux ne la précipitera que très lentement.

L'utilisation d'une phase supérieure constituée par un non solvant doux permet à la capsule de pénétrer dans le non solvant fort sans être déformée.

Dans le cas où le non solvant fort utilisé est une solution aqueuse, le remplacement du solvant de la solution polymère par de l'eau conduit à la formation d'un hydrogel, ce qui est favorable à l'obtention de bonnes qualités de biocompatibilité.
Le fonctionnement du dispositif ainsi décrit est le suivant.

Le fluide vecteur est mis en circulation à partir du réservoir 5 grâce à la pression exercée par de l'air provenant de la canalisation 15. La pression d'air est commandée par le dispositif 17 pour fournir un débit approprié. Afin de favoriser l'écoulement du fluide vecteur dans la canalisation 1 en direction de la buse 6, et éviter son écoulement vers le réservoir 2, il est possible de clamper le conduit 1 sur la portion comprise entre l'intersection 3 et le réservoir 2 ou de produire dans cette portion de conduit une augmentation de la pression.

Lorsque le fluide vecteur atteint le réservoir 11, on commande le dispositif 17 pour alimenter le réservoir 8 en air comprimé, afin d'induire l'écoulement de solution polymère dans la buse 6, par l'intermédiaire du conduit 7. On ajuste alors la distance d par déplacement vertical du conduit 1 dans la buse 6, de manière à régler l'épaisseur de l'enveloppe de la capsule. On commande ensuite le dispositif 17 pour mettre en circulation la substance à encapsuler dans le conduit 1. Les pressions d'air à l'intérieur des réservoirs 2, 5 et 8 sont commandées en fonction des débits souhaités pour la substance à encapsuler, le fluide vecteur et la solution polymère.

Le débit du fluide vecteur (ainsi que sa viscosité) conditionne la taille des segments de substance à encapsuler ; pour un débit de fluide vecteur donné, le débit de la substance à encapsuler conditionne l'écartement existant entre deux segments de substance à encapsuler : plus le débit augmente, plus l'écartement entre deux segments est faible. Le débit d'alimentation de la buse 6 en solution polymère est ensuite réglé pour obtenir la formation d'une goutte de solution polymère pour chaque segment de substance à encapsuler.

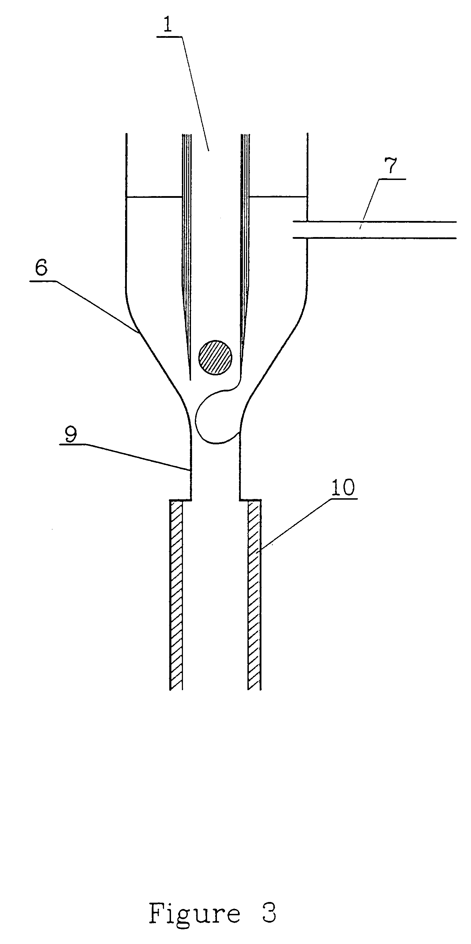

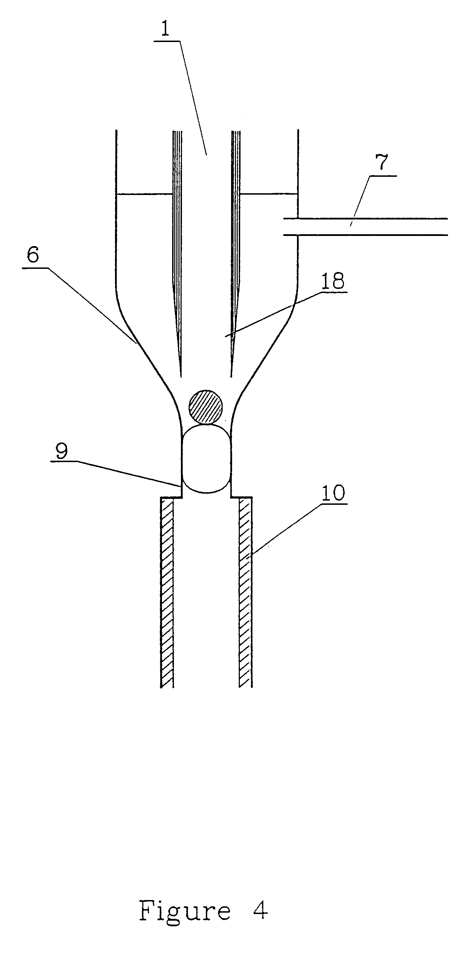

Ainsi que cela est représenté sur la figure 3, lorsque la synchronisation est réalisée entre le flux de substance à encapsuler et la solution polymère, grâce à la paroi effilée 18 de l'extrémité du conduit 1, la solution polymère est extrudée sous forme de goutte qui tombe à l'intérieur du canal de constriction 9 (figure 4) suivie de la substance à encapsuler. La longueur du canal 9 est choisie juste suffisante pour provoquer un allongement de la goutte.

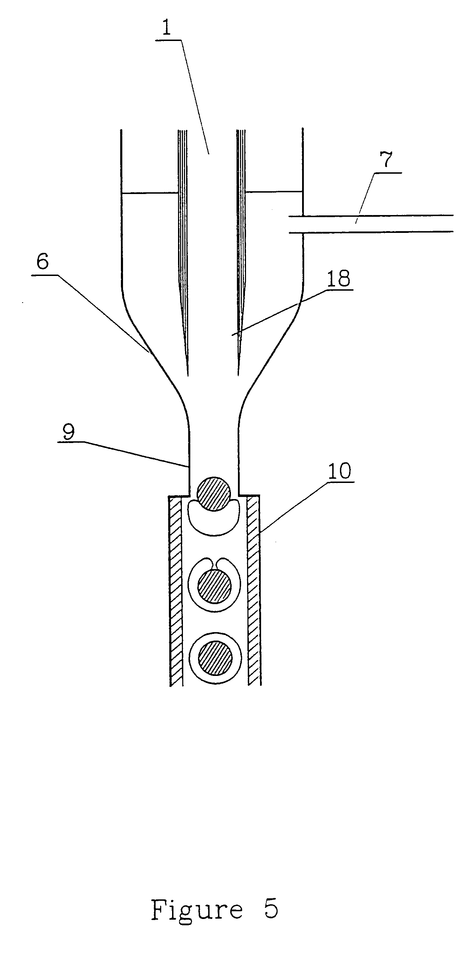

La forme allongée de la goutte est favorable à ce que la goutte prenne ensuite la forme d'un haricot de révolution (figure 5) lorsque, en sortie du canal 9, elle débouche dans un conduit 10 de section plus importante. Lors de la progression de la goutte et de la substance à encapsuler dans le conduit 10, l'affinité qu'elles présentent l'une pour l'autre permet d'écarter le fluide vecteur, ce qui conduit à l'enrobage de la substance à encapsuler par la solution polymère.

Afin de faciliter cet enrobage, il est possible de régler l'écoulement de la solution polymère pour que la goutte de solution polymère formée se détache de la paroi 18 au moment même où un segment de substance à encapsuler parvient à l'extrêmité du conduit 1 (ainsi que représenté sur la figure 3) ; ceci permet d'obtenir immédiatement une mise en contact de la substance à encapsuler avec la solution polymère, ce qui favorise ensuite l'opération d'enrobage.

Selon une caractéristique de l'invention, la capsule formée est ensuite refroidie lors de son écoulement à l'intérieur du conduit 10 grâce à un dispositif de réfrigération constitué essentiellement par une double enveloppe 12 à l'intérieur de laquelle circule un liquide de refroidissement tel que de l'éthylène glycol. Le liquide de refroidissement circule à contre courant par rapport au sens d'écoulement des capsules formées.

L'abaissement de la température protège la substance à encapsuler des effets néfastes possibles du solvant présent dans la solution polymère ; il facilite également la pénétration sans déformation de la capsule formée dans le non solvant du réservoir 11.

De façon avantageuse, on refroidit jusqu'à atteindre une température inférieure à la température de gélification de la solution polymère ; ainsi, l'enveloppe enrobant la substance à encapsuler passe de l'état de solution à l'état de gel thermoréversible.
Ce passage par l'état de gel thermoréversible permet, après la phase d'échange du solvant par un non solvant présent dans le réservoir 11, d'obtenir un gel irréversible dont la structure est homogène et la surface parfaitement lisse ; on obtient ainsi une membrane enveloppant la capsule, dont la structure ne présente ni doigt ni peau.

Le bain du réservoir 11 est avantageusement maintenu à une température inférieure à la température ambiante de manière à éviter tout réchauffement brutal de la capsule qui conduirait au passage de l'état de gel thermoréversible à celui de solution et également pour compenser une éventuelle exothermicité de l'échange solvant/non solvant.

Après l'opération d'échange du solvant par du non solvant, on obtient des capsules dont l'enveloppe est constituée par un gel non thermoréversible. Ces capsules sont lavées puis conservées dans un milieu approprié avant leur utilisation.

De façon alternative à ce qui vient d'être décrit, il est possible de fabriquer des macrocapsules se présentant sous forme de fibres creuses, dont la paroi est constituée d'un hydrogel d'acrylonitrile et de méthallylsulfonate de sodium.

Ces macrocapsules sont obtenues par coextrusion du matériel à encapsuler et d'une solution polymère contenant un copolymère d'acrylonitrile et de méthallylsulfonate de sodium, du chlorure de sodium et du diméthylsulfoxide, poursuivi par une étape de coagulation dans un bain précipitant à température ambiante. Les fibres obtenues ont une paroi de 100 µm d'épaisseur et un diamètre interne de 450 µm.

### Exemple 1

On décide d'encapsuler des cellules RIN en suspension dans une solution aqueuse à 0,9 % de NaCl et à 1 % d'albumine. Les cellules RIN sont des cellules tumorales de souris aptes à sécréter de l'insuline lorsqu'elles sont stimulées par de l'arginine.

Dans le dispositif d'encapsulation utilisé, les conduits capillaires 1 et 4 sont constitués par un tube en polytétrafluoroéthylène de section droite circulaire de diamètre interne 0,7 mm et de diamètre externe 1,1 mm. Le canal de constriction 9 a une longueur de 3 mm et un diamètre interne de 0,9 mm. Le conduit réfrigéré 10 est constitué par un tube de 35 cm de longueur, de diamètre interne 1,1 mm et de diamètre externe 1,7 mm. Afin de permettre un réglage précis du débit de solution polymère au moyen d'une variation de la pression d'air à l'intérieur du réservoir 8, on choisit un tube 7 d'amenée de la solution polymère dont le diamètre est inférieur à 5 mm. L'angle d'ouverture α de la buse conique 6 est d'environ 45° et la distance entre l'extrémité 18 de la paroi effilée et la buse 6 est d'environ 0,2 mm.

Le fluide vecteur utilisé est un mélange constitué par :
. 78 % d'huile silicone à 170 ppm de groupements hydroxyles, de viscosité égale à 3 cPo ;
. 17 % d'huile silicone à 1010 ppm de groupements hydroxyles, de viscosité égale à 3 500 cPo ;
. 5 % d'hexadécane.

Le mélange obtenu comprend 306 ppm de groupements hydroxyles et présente une viscosité de 27 cPo ; il est mis en circulation dans le conduit 4 à un débit de 0,30 ml/min.

La solution polymère utilisée est constituée de :
. 6 % d'un copolymère d'acrylonitrile et de méthallylsulfonate de sodium ;
. 3 % de solution aqueuse de chlorure de sodium à 9 g/l ;
. 91 % de diméthylsulfoxide.

La température de gélification de cette solution se situe autour de 9°C.

Le débit d'alimentation de la buse 6 en solution polymère est de 0,07 ml/min.

La substance à encapsuler constituée par les cellules RIN en suspension dans une solution saline à une concentration d'environ 10⁶ cellules/ml est mise en circulation dans le conduit 1 avec un débit de 0,01 ml/min. Les réservoirs 2, 5 et 8 sont à température ambiante.

La coextrusion est réalisée à 24°C. La réfrigération du conduit 10 est réalisée grâce à la circulation à contre-courant dans la double enveloppe 12 d'un mélange éthylène-glycol/eau (40/60) dont la température de consigne en sortie du dispositif de réfrigération 13 est de 1°C.

Le bain du réservoir 11 comprend une phase inférieure constituée par une solution aqueuse de chlorure de sodium à 9 g/l et une phase supérieure de plus petit volume constituée par un mélange constitué par 70 % en volume d'éthanol, 4 % de butanol, 1 % d'isopropanol, 25 % d'eau, auquel on ajoute quelques gouttes de bleu de méthylène. Le bain est à température ambiante.

L'interface entre les deux phases présente un gradient de concentration alcoolique qui favorise la pénétration sans déformation des capsules.

Les capsules obtenues sont parfaitement sphériques avec un diamètre externe compris entre environ 800 µm et 900 µm. L'enveloppe est translucide et a une épaisseur constante de 100 µm. Elle est constituée d'un hydrogel composé de 86 % d'eau et de 14 % de copolymère d'acrylonitrile et de méthallylsulfonate de sodium.

Après lavage dans une solution aqueuse de NaCl à 9 g/l, les capsules sont mises en culture dans du RPMI contenant 10 % de sérum de veau foetal.
Au bout de 19 jours, l'observation microscopique montre une multiplication des cellules RIN à l'intérieur de la capsule.

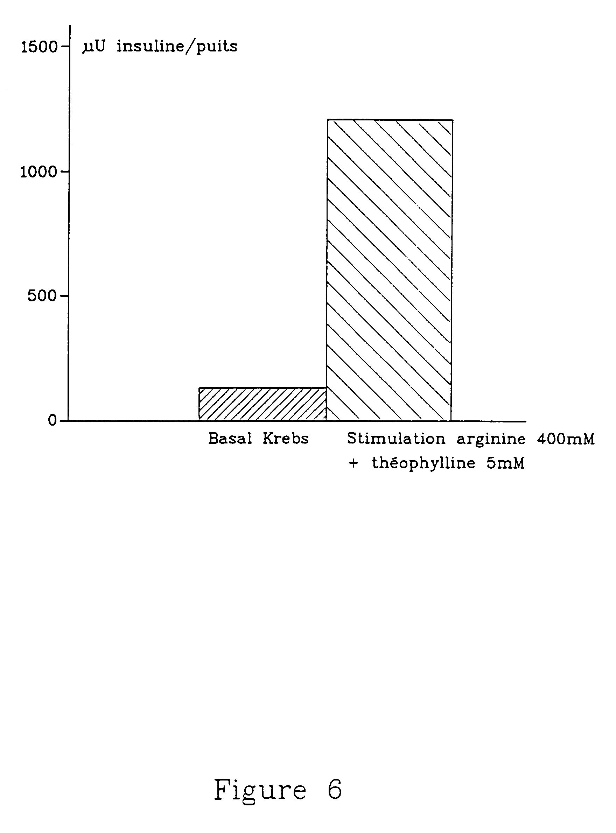

Un test de stimulation à l'arginine démontre la fonctionnalité des cellules RIN encapsulées. En effet, 15 à 27 capsules sont placées dans un puits. Le milieu de culture est remplacé par une solution de Krebs. Une première incubation témoin est réalisée permettant de connaître le taux basal d'insuline. Cette solution est ensuite remplacée par une solution de Krebs "stimulante", contenant de l'arginine et de la théophyline. L'expérience est réalisée sur 10 puits. Les résultats de dosage de l'insuline pour chaque puits sont illustrés sur la figure 6, et montrent une sécrétion d'insuline lors de la stimulation des capsules.

Ainsi, on peut en conclure que les cellules RIN restent vivantes et fonctionnelles à l'intérieur de la capsule dont l'enveloppe est perméable aux nutriments, à l'arginine et à l'insuline.

### Exemple 2

La substance à encapsuler est constituée dans ce cas par une solution d'insuline à 40 UI/ml. Le dispositif d'encapsulation, la composition de la solution polymère, du bain d'échange solvant/non solvant ainsi que les températures de mise en oeuvre du procédé sont les mêmes que dans l'exemple 1.

Le fluide vecteur est ici un mélange constitué par :
. 17 % d'huile silicone à 2500 ppm de groupements hydroxyles, dont la viscosité est de 750 cPo ;
. 83 % d'huile silicone à 170 ppm de groupements hydroxyles, dont la viscosité est de 3 cPo.

Le mélange obtenu comporte environ 570 ppm de groupements hydroxyles et présente une viscosité de 13,4 cPo.

Les débits de circulation sont les suivants :
- solution d'insuline : 0,008 ml/min
- huile : 0,47 ml/min
- solution polymère : 0,06 ml/min.

Les capsules obtenues sont parfaitement sphériques et présentent un diamètre externe compris entre environ 800 µm et 900 µm avec une enveloppe translucide dont l'épaisseur est constante et d'environ 100 µm.

### Exemple 3

La substance à encapsuler est constituée ici par des îlots de Langerhans en suspension dans une solution aqueuse de NaCl à 9 g/l. La composition de la solution polymère, du fluide vecteur et du liquide d'échange solvant/non solvant est la même qu'à l'exemple 2.

Les débits de circulation sont les suivants :
- suspension d'îlots : 0,019 ml/min
- huile : 0,366 ml/min
- solution polymère : 0,053 ml/min

La coextrusion des îlots et de la solution polymère est effectuée à température ambiante.

La température de consigne du liquide de refroidissement en sortie du dispositif de réfrigération 13 est de -6°C. Le bain d'échange solvant/non solvant est à une température variant de 5°C à 15°C.

Les capsules obtenues sont des sphères parfaites comportant un noyau constitué par des îlots de Langerhans en suspension dans la solution saine entourés par une enveloppe translucide constituée par un hydrogel d'acrylonitrile et de méthallylsulfonate de sodium.
Le diamètre externe de la capsule est compris entre environ 800 µm et 900 µm ; l'épaisseur de la capsule est d'environ 100 µm.
Les capsules sont conservées dans du milieu de culture (RPMI) contenant 10 % de sérum de veau foetal.

### Exemple 4

On réalise dans cet exemple l'encapsulation de bactéries (Escherichia coli) en suspension dans un bouillon de culture Trypcase-Soja (TS).

Les conditions d'encapsulation sont les mêmes que dans l'exemple 1, à l'exception des débits d'écoulement qui sont :
- pour la suspension de bactéries : 0,0145 ml/min
- pour l'huile : 0,47 ml/min
- pour la solution polymère : 0,06 ml/min

Les capsules obtenues sont des sphères parfaites formées par une enveloppe entourant un noyau de 650 µm de diamètre constitué par la suspension de bactéries. Elles sont mises en culture dans du bouillon TS à 37°C.

L'observation des capsules après 24 h, puis 8 jours de mise en culture montre une multiplication des bactéries sans éclatement de l'enveloppe.

### Exemple 5

On fabrique des microcapsules remplies de liquide physiologique selon la méthode décrite à l'exemple 1, afin d'étudier leur biocompatibilité vis-à-vis du site d'implantation.

Les capsules sont ensuite implantées chez le rat dans le tissu sous-cutané. Après une période de 3 semaines, on observe que les capsules sont agglutinées et forment une structure organoïde qui peut être énucléée. L'étude de la réaction tissulaire autour des capsules montre une diminution significative de l'épaisseur de la couche cellulaire observée à 2 mois par rapport à celle observée à 3 mois, ce qui est la conséquence de la disparition de la couche de macrophages adhérente à la capsule et de la régression du tissu conjonctif néoformé lors de la réaction inflammatoire.
En outre, les espaces laissés libres entre les capsules sont parcourus à 2 mois par un réseau capillaire, favorable aux échanges. L'observation faite à 6 mois montre que l'épaisseur de la fibrose est identique à celle observée à 2 mois.
Des capsules sont également implantées dans la cavité péritonéale de rats et conduisent à une réaction similaire à celle observée en sous-cutané.

### Exemple 6

On fabrique des fibres creuses remplies d'îlots de Langerhans, par coextrusion d'une suspension d'îlots et d'une solution polymère constituée de :
- 10 % d'un copolymère d'acrylonitrile et de méthallylsulfonate de sodium,
- 5 % de solution aqueuse de chlorure de sodium à 9 g/l,
- 85 % de diméthylsulfoxide.

La coextrusion est réalisée au moyen d'une filière en acier comprenant un canal interne dont le diamètre est d'environ 330 µm entouré d'un canal annulaire externe de 845 µm, l'épaisseur de la paroi entre les 2 canaux étant de 130 µm.
Le débit d'extrusion de la suspension d'îlots est de 0,16 ml/min et celui de la solution polymère est de 3 ml/min.
La fière est placée à environ 20 cm au-dessus d'un bain de liquide précipitant constitué par une solution aqueuse de chlorure de sodium à 9 g/l à température ambiante. En sortie de filière, la fibre coextrudée tombe dans le bain de précipitation où elle est coagulée. La paroi de la fibre est constituée d'un hydrogel composé de 80 % d'eau et de 20 % de copolymère d'acrylonitrile et de méthallylsulfonate de sodium.

### Exemple 7

Les fibres obtenues selon l'exemple 6 ont été implantées dans le tissu sous-cutané et dans la cavité péritonéale de rats. La réaction tissulaire observée est comparable à celle décrite à l'exemple 5, et est ici aussi stable après 2 mois d'implantation.
En outre, des fibres explantées de la cavité peritonéale 3 semaines après leur implantation ont été mises en culture puis stimulées.
Certaines des fibres testées ont montré leur capacité à répondre à une solution stimulante de glucose par une sécrétion augmentée d'insuline.

Ainsi, on peut en déduire que les îlots de Langerhans restent vivants et fonctionnels à l'intérieur de la fibre dont l'enveloppe est perméable aux nutriments, à l'insuline et au glucose.

## Revendications

1. Capsule implantable comprenant :
- une substance biologiquement active ;
- une enveloppe entourant la substance biologiquement active, l'enveloppe étant constituée par un hydrogel d'acrylonitrile et de méthallylsulfonate de sodium contenant au moins 80% d'eau.

2. Capsule implantable, selon la revendication 1, caractérisée en ce qu'elle possède une forme sphérique de diamètre sensiblement compris entre 800 µm et 900 µm et en ce que l'enveloppe a une épaisseur constante sensiblement égale à 100 µm.

3. Capsule implantable selon une des revendications 1 ou 2, caractérisée en ce que la substance comprend des îlots de Langerhans ou des cellules β de pancréas.

4. Capsule implantable selon la revendication 3, caractérisée en ce que l'enveloppe est perméable à l'insuline.

5. Capsule implantable selon une des revendications 1 ou 2, caractérisée en ce que la substance comprend des hépatocytes.

6. Capsule implantable selon une des revendications 1 à 5, caractérisée en ce que l'enveloppe est perméable aux nutriments nécessaires à la substance encapsulée.

## Claims

1. An implantable capsule comprising:
- a biologically active material;
- a shell encapsulating the biologically active material, the shell consisting of a hydrogel of acrylonitrile and of sodium methallylsulphonate, said hydrogel containing at least 80 % of water.

2. An implantable capsule according to claim 1, characterized in that it has a spherical shape with a diameter substantially comprised between 800 µm and 900 µm and in that the shell has a constant thickness substantially equal to 100 µm.

3. An implantable capsule according to claim 1, characterized in that the biologically active material includes islets of Langerhans or pancreatic β cells.

4. An implantable capsule according to claim 3, characterized in that the shell is permeable to insulin.

5. An implantable capsule according to one of the claims 1 or 2, characterized in that the biologically active material includes hepatocytes.

6. An implantable capsule according to one of claims 1 to 5, characterized in that the shell is permeable to the nutrients required by the encapsulated biologically active material.

## Patentansprüche

1. Implantationskapsel mit :
- einer biologisch aktiven Substanz ;
- einer Hülle, die die biologisch aktive Substanz umgibt, wobei die Hülle aus einem Hydrogel aus Acrylnitril und Natriummethallylsulfonat besteht und einen Wassergehalt von mindestens 80 % aufweist.

2. Implantationskapsel nach Anspruch 1, dadurch gekennzeichnet, daß sie kugelförmig ist und einen Durchmesser im wesentlichen zwischen 800 mm und 900 mm aufweist und daß die Hülle eine gleichmäßige Dicke von im wesentlichen 100 mm aufweist.

3. Implantationskapsel nach einem der Ansprüche 1 oder 2, dadurch gekennzeichnet, daß die Substanz Langerhans-Inseln oder β-Zellen der Bauchspeicheldrüse enthält.

4. Implantationskapsel nach Anspruch 3, dadurch gekennzeichnet, daß die Hülle insulindurchchlässig ist.

5. Implantationzkapsel nach einem der Ansprüche 1 oder 2, dadurch gekennzeichnet, daß die Substanz Hepatocyten enthält.

6. Implantationskapsel nach einem der Ansprüche 1 bis 5, dadurch gekennzeichnet, daß die Hülle für die für die eingekapselte Substanz erforderliche Nahrung durchlässig ist.
